# EUROPÄISCHE PATENTSCHRIFT

(11) **EP 3 068 868 B1**
(45) Veröffentlichungstag und Bekanntmachung des Hinweises auf die Patenterteilung: **06.05.2020**
(21) Anmeldenummer: 14812139.5
(22) Anmeldetag: 11.11.2014
(51) Int. Cl.: C12N 1/00

(54) **VERFAHREN ZUR HERSTELLUNG VON BIOGENEN STOFFEN**
METHOD FOR PRODUCING BIOGENIC SUBSTANCES
PROCÉDÉ DE PRODUCTION DE SUBSTANCES BIOGÈNES

(30) Priorität: 12.11.2013 EP 13192606
(43) Veröffentlichungstag der Anmeldung: 21.09.2016
(73) Patentinhaber: Brandenburgische Technische Universität Cottbus-Senftenberg, 01968 Senftenberg (DE)
(72) Erfinder: SCHEIBNER, Katrin, 01968 Senftenberg (DE); KÜPPER, Jan-Heiner, 01968 Grosskoschen/ OT Kleinkoschen (DE); SCHMIDTKE, Kai-Uwe, 07747 Jena (DE); MIETHBAUER, Sebastian, 07743 Jena (DE); HERZOG, Natalie, 03046 Cottbus (DE)
(74) Vertreter: Simandi, Claus
(86) Internationale Anmeldenummer: PCT/EP2014/074278
(87) Internationale Veröffentlichungsnummer: WO 2015/071264

(56) Entgegenhaltungen:
- WO-A1-2010/123357
- WO-A2-02/40995
- WO-A2-2009/007678
- CN-A- 103 374 573
- M. A. SELLS ET AL: "Production of hepatitis B virus particles in Hep G2 cells transfected with cloned hepatitis B virus DNA.", PROCEEDINGS NATIONAL ACADEMY OF SCIENCES PNAS, vol. 84, no. 4, 1 February 1987 (1987-02-01), pages 1005-1009, XP055379467, US ISSN: 0027-8424, DOI: 10.1073/pnas.84.4.1005
- ALAM T ET AL: "Glucose-regulated insulin production in hepatocytes", TRANSPLANTATION, WILLIAMS AND WILKINS, GB, vol. 74, no. 12, 27 December 2002 (2002-12-27), pages 1781-1787, XP002685524, ISSN: 0041-1337, DOI: 10.1097/01.TP.0000039333.11465.D9

## Beschreibung

Gegenstand der Erfindung ist ein Verfahren zur Herstellung von biogenen Stoffen.

Es besteht ein hohes Bedürfnis an verbesserten Herstellungsverfahren für biogene Stoffe. Besonders bevorzugt ist die Herstellung solcher biogenen Stoffe, die bei der Biotransformation oder Metabolisierung in Leberzellen (Hepatozyten) bzw. in der Leber entstehen.

Die Leber ist ein zentrales Entgiftungsorgan des Stoffwechsels. Leberzellen (Hepatozyten) repräsentieren 70-80% aller Zellen in der Leber und verfügen über die wichtigen physiologischen Leberfunktionen (Elaut et al. (2006)).

Die Leber verwendet die Biotransformation oder Metabolisierung um aufgenommene Substanzen (z.B. Medikamente, Toxine, Naturstoffe) ausscheiden zu können bzw. zu entgiften. Für die Biotransformation wichtig sind insbesondere die Phase-I-Enzyme des Cytochrom-P-450 (CYP450) Systems. Es handelt sich bei den CYP450-Enyzmen um Oxidoreduktasen, die einen oxidativen Abbau bzw. Metabolisierung zahlreicher Substanzen wie u.a. auch Arzneistoffe bewirken. Von den zahlreichen CYP450-Isoenzymen mit unterschiedlicher Substratspezifität, die es beim Menschen gibt, sind alleine die Isoenzyme CYP1A2, -2C9, -2C19, -2D6, - 2E1 und -3A4 für ca. 90 % sämtlicher oxidativer Metabolisierungen von Medikamenten verantwortlich (Arimoto (2006); Shimada *et al.* (1994); Lamb *et al.* (2007)). In vielen Fällen erhalten eine Vielzahl von Medikamenten erst durch diese biochemischen Veränderungen ihre curative Wirksamkeit oder verursachen gar giftige Metaboliten mit unerwünschten Arzneimittelwirkungen (Chang et al. 2007).

Diese Stoffe sind vor allem als Abbauprodukte der Leber interessant und bedürfen der weiteren Untersuchung. Hierzu sind solche Lebermetaboliten in ausreichender Menge herzustellen, insbesondere weisen diese biogenen Stoffe eine hohe regioselektive und stereoselektive Spezifität auf. Solche Stereoisomere weisen regio- als auch stereoselektive Modifkationen auf, die charakteristisch für biotransformatorische Enzyme sind, wie nachfolgend beschrieben.

Für die Biotransformation oder Metabolisierung in Leberzellen sind Phase I Enzyme beschrieben, insbesondere des Cytochrom-P-450 (CYP450) Systems, so genannte Oxidoreduktasen. Weiterhin sind Phase II Enzyme relevant, wie z.B. N-Acetyltransferasen [NATs], UDP-Glucuronosyltransferasen, Sulfotransferasen. Für die Beurteilung der Lebertoxizität von Substanzen ist die Aktivität der Phase-I-Enzyme und Phase-II-Enzyme sowie weiterer Leberfunktionen von entscheidender Bedeutung.

Beachtlich ist weiterhin, dass solche biotransformatorische Enzyme evolutionär bedingt ebenfalls in anderen Organismen, wie Pilze und Bakterien vorkommen können.

WO 2008/119780 A2 beschreibt ein Verfahren zur enzymatischen Hydroxylierung nicht-aktivierter Kohlenwasserstoffe, insbesondere aromatischer Ringe nicht-aktivierter Kohlenwasserstoffmoleküle (beispielsweise die selektive Umsetzung von Naphthalin zu 1-Naphthol) durch Verwendung von pilzlichen Peroxidasen aus Basidiomyceten der Familie Bolbitiaceae (z.B. Agrocybe sp.) zur Herstellung von Pharmazeutika, Terpenen, Steroiden oder Fettsäuren.

DE102008034829 A1 offenbart ein einstufiges, enzymatisches Verfahren zur regioselektiven Hydroxylierung von 2-Phenoxypropionsäure zu 2-(4-Hydroxyphenoxy)propionsäure. Die enantioselektive und regioselektive Monohydroxylierung von 2-Phenoxypropionsäure zu 2-(4-Hydroxyphenoxy)propionsäure durch isolierte Biokatalysatoren (in vitro) kann ebenfalls durch ein stabiles extrazelluläres Pilzenzym, die Agrocybe-aegerita-Peroxygenase (AaP), 2-Phenoxypropionsäure hoch regioselektiv zu 2-(4-Hydroxyphenoxy)propionsäure und bevorzugt zu dessen (R)-Enantiomer umgesetzt werden.

Im Stand der Technik ist die Herstellung von biogenen Stoffen mittels Koppelung von Enzymen und Leberzellen nicht beschrieben.

Im Stand der Technik ist nachteilig, dass biogene Stoffe nicht in ausreichender Ausbeute und Vielfalt hergestellt werden können. Zudem werden im Stand der Technik zumeist Vorläufermoleküle synthetisiert. Oftmals sind zusätzlich halbsynthetische Verfahren erforderlich, so dass die erforderlichen weiteren regio- und stereoselektiven Modifikationen in die Substanz(en) aufwändig eingeführt werden können, die für Metabolite aus der Leber entscheidend sind.

Die Erfindung betrifft daher die Herstellung von biogenen Stoffen, die nach einem neuen Verfahren hergestellt werden.

Erfindungswesentlich ist, dass die Synthese von biogenen Stoffen unter Einsatz von Enzymen in Kombination mit einem Leberzellsystem erfolgt.

Gegenstand der Erfindung ist daher ein Verfahren zur Herstellung von biogenen Stoffen, dadurch gekennzeichnet, dass
a) mindestens ein Ausgangsstoff mit
b) mindestens einem Enzym versetzt wird und das erhaltene Produkt aus b) mit
c) mindestens einer Leberzelle versetzt wird,
   oder
b') mindestens eine Leberzelle versetzt wird und das erhaltene Produkt aus b) mit
c') mindestens einem Enzym versetzt wird,
   und
d) mindestens ein biogener Stoff isoliert wird.
   (nach- und vorstehend "erfindungsgemäßes Verfahren")

Überraschender Weise lassen sich kostengünstig leberrelevante biogene Stoffe in hoher Ausbeute kontinuierlich oder diskontinuierlich herstellen, wobei vorteilhaft neue stereoselektive Verbindungen (Metaboliten) erhalten werden können.

Die Ausgangstoff(e) oder Edukt(e) enthalten mindestens eine chemische Substanz, ein Substanzgemisch, insbesondere einen Arznei- oder Wirkstoff. Die chemischen Substanzen sind vorzugsweise ein organisches Molekül, welches neben Kohlenstoff (C) und Wasserstoff (H) Heteroatome enthalten können, wie Sauerstoff (0), Stickstoff (N), Schwefel (S) oder Phosphor (P). Die chemischen Substanzen können lineare und/oder ringförmige Kohlenstoffketten samt Heteroatomen aufweisen. Bevorzugt sind organische Moleküle mit weniger als 1.000 g/mol, insbesondere weniger als 750 g/mol, weniger als 500 g/mol, weniger als 250 g/mol. Weiterhin ist bevorzugt, dass mindestens eine chemische Substanz mindestens ein chirales Kohlenstoffatom enthält.

Der Begriff "biogene Stoffe" im Sinne dieser Erfindung bedeutet, dass das erfindungsgemäße Verfahren die Herstellung von Stoffen bzw. von chemischen Substanzen und zwar vorzugsweise von neuen Stereoisomeren erlaubt. Die chemischen Substanzen sind vorzugsweise organische Moleküle, welche neben Kohlenstoff (C) und Wasserstoff (H) Heteroatome enthalten können, wie Sauerstoff (0), Stickstoff (N), Schwefel (S) und Phosphor (P). Die chemische Substanz kann lineare und/oder ringförmige Kohlenstoffketten samt Heteroatomen aufweisen. Bevorzugt sind organische Moleküle mit weniger als 1.000 g/mol, insbesondere weniger als 750 g/mol, weniger als 500 g/mol, weniger als 250 g/mol.

Die Ausgangstoffe als auch biogene Stoffe können z.B. mittels kombinierten analytischen Verfahren, wie GC/LC-MS, IR, NMR hinreichend analysiert werden und ggfs. einer Strukturaufklärung unterzogen werden. Insbesondere die Massenspektroskopie erlaubt eine hinreichende schnelle Identifizierung der besagten Stoffe, wie z.B. Molmasse, etc.

Ein "Enzym" im Sinne dieser Erfindung ist ein Protein, welches eine oder mehrere biochemische Reaktionen katalysieren kann. Ein erfindungsgemäßes Enzym ist in der Lage aus Ausgangsstoffen (Edukten) - einem Substrat - ein erstes (Enzym)Produkt oder Produkte zu erhalten. Leberspezifische Enzyme sind in einer Leberzelle enthalten.

In einer bevorzugten Ausführungsform werden die Enzyme ausgewählt aus der Gruppe der Oxidoreduktasen (EC 1.x.x.x), insbesondere Monooxygenasen, Dioxygenasen, Oxidasen, Dehydrogenasen, Reduktasen und Peroxygenasen. Besonders bevorzugt sind Peroxygenasen. Weiterhin sind Enzyme der Biotransformation geeignet, insbesondere Phase-I-Enzyme und/oder Phase-II-Enzyme. Darüber hinaus sind Esterasen (EC 3.1.x.x), Hydrolasen (EC 3.x.x.x) und Transferasen (EC 2.x.x.x) geeignet. Entsprechende Enzyme können anhand der bekannten EC-Enzymklassen oder Nomenklatur zugewiesen werden.

Weiterhin ist bevorzugt, dass das Enzym aus b) und c') im Gegensatz zu der Leberzelle aus einem anderen Organismus stammt.

In einer bevorzugten Ausführungsform wird ein erstes Enzym aus Pilzen, Hefen, Algen oder Bakterien erhalten und die eingesetzten Leberzellen sind vorzugsweise humane Hepatozyten. Pilze sind jedoch erfindungsgemäß bevorzugt.

Die Enzyme können nach bekannten Verfahren aus den Organismen isoliert und aufgereinigt werden. Weiterhin können solche Enzyme rekombinant in einem Wirt hergestellt werden.

"Leberzelle" im Sinne dieser Erfindung bedeutet, dass die Zelle mindestens Enzyme zur Biotransformation aufweist, insbesondere Phase-I-Enzyme und/oder Phase-II-Enzyme und folglich hinreichend eine Leberfunktion ausführt. Die Leberzelle ist vorzugsweise eine solche, die aus humanen Leberzellen oder Säugerleberzellen erhalten werden kann. Solche Hepatozyten können z.B. aus den Lehren gemäß WO2009/030217A2 und WO2012/045731A1 bereitgestellt werden, umfassend genetisch veränderte Hepatozyten, welche z.B. ein Proliferationsgen aufweisen, so genannte proliferierende Leberzellen.

Ebenfalls sind erfindungsgemäß genetisch veränderte Leberzellen umfasst, die (transgen) beliebig verändert sein können. Beispielsweise können solche Leberzellen mittels viralen Vektoren (z.B. Lentiviren, z.B. DE 69830663 T2, EP 1175436 B1) hergestellt werden. Die Herstellung solcher genetisch veränderter Leberzellen ist dem Fachmann bekannt, vorzugsweise können Phase I und Phase II Enzyme ebenfalls rekombinant vorliegen. Weiterhin können Leberzelllinien erfindungsgemäß umfasst sein, die dem Fachmann bekannt sind, wie nicht abschließend käuflich erwerblich (www.cell-lines-service.de) Chang-Liver (Humane Leber Zell-Linie), Hep-G2 (Humane Hepatom Zell-Linie), HuH-7 (Humane Hepatoblastom Zell-Linie), PLC-PRF-5 (Humane Hepatom Zell-Linie), SK-HEP-1 (Humane Leber Adenokarzinom Zell-Linie) als auch z.B. Fa2N-4, Hep3B, BC2, HepaRG. Erfindungsgemäß können die Leberzellen ebenfalls im Zellverband auftreten.

Zu den erfindungsgemäßen relevanten Phase I Enzymen zählen insbesondere Cytochrom P450 System, Alkohol Dehydrogenasen, Aldehyd Dehydrogenasen, Peroxidasen, Glutathionperoxidase, Esterasen, Hydrolasen.

Zu den erfindungsgemäßen relevanten Phase 1I Enzymen zählen insbesondere Glucuronyltransferasen, Sulfotransferasen, Glutathion-S-Transferase, Methyltransferasen, Aminotransferasen / Transaminasen, Acetyltransferasen.

Die Verfahrensschritte b) (b') und c) (c') können in einer Ein-Topfvorrichtung durchgeführt werden. Eine solche Ein-Topfvorrichtung ist bevorzugt als Bioreaktor ausgestaltet.

In einer weiteren bevorzugten Ausführungsform sind die Verfahrensschritte b) (b') und c) (c') voneinander räumlich getrennt, z.B. mittels zwei oder mehreren voneinander getrennten Bioreaktoren. Dies kann ebenfalls in der Weise erfolgen, dass ein Bioreaktor zwei oder mehr Schichten aufweist, wobei jeweils in einer Schicht mindestens ein erfindungsgemäßer o.g. Verfahrensschritt durchgeführt wird. Bevorzugt ist jedoch der Einsatz von zwei oder mehreren unabhängigen Bioreaktoren, die vorzugsweise miteinander gekoppelt sein können.

Daher betrifft die Erfindung ein erfindungsgemäßes Verfahren, wobei mindestens ein Bioreaktor, vorzugsweise zwei oder mehrere Bioreaktoren zur Ausführung der Verfahrensschritte b) (b') und c) (c') eingesetzt werden, und mindestens ein Schritt b) (b') und c) (c') auch wiederholt oder nachgeschaltet werden kann.

Die Bioreaktoren können mit Rührer und anderen üblichen Hilfsmitteln ausgestattet sein. Geeignete Bioreaktoren zur Kultivierung sind solche wie Hohlfaser-Bioreaktoren, Rührkesselreaktor (Fermenter), Wirbelschichtreaktor (Aggregate oder poröse Trägermaterialien), Festbettreaktoren mit Hohlfaser- bzw. Flachbettmembranen (Verfahren ist Tangentialflussfiltration), Plug-Flow-Reactor, Spinner Flaschen Kultivierung (siehe Horst Chmiel (Hrsg.) Bioprozesstechnik (2011), Spektrum Akademischer Verlag, Heidelberg). Die Bioreaktoren können ein Medium, Kulturmedium, Kulturflüssigkeit enthalten, insbesondere solche Medien, die für Leberzellen geeignet sind. Geeignete Zellkulturen und Kulturmedien sind dem Fachmann einschlägig bekannt und käuflich erwerblich (siehe Beispiele).

In einer weiteren bevorzugten Ausführungsform sind die Bioreaktoren miteinander gekoppelt. Die Kopplung kann beispielsweise durch eine beliebige Verbindung zweier oder mehrerer Bioreaktoren erfolgen, z.B. entlang einem Flussgradienten, wobei Medium, Kulturflüssigkeit oder Überstand von einem Bioreaktor zu einem anderen Bioreaktor gelangt.

Zur Durchführung der Erfindung können die Enzyme gemäß b) und c') in der Weise eingesetzt werden, dass die Enzyme in einem Enzym-Membranreaktor oder immobilisiert auf einem beliebigen Träger vorliegen. Weiterhin geeignet sind Scaffolds, Mikrosomen oder zellfreie Systeme. Ebenfalls können die Enzyme mittels Kollagen oder Gelatine fixiert werden.

Die Isolierung der erhaltenen biogenen Stoffe (Down-Stream-Prozess) oder Zwischenprodukte kann mittels Aufschließen der Leberzellen oder Aufreinigung aus dem Überstand erfolgen (Schritt d.)). Die erhaltenen Produkte und Verbindungen können z.B. mittels LC/MS identifiziert werden.

Nachfolgende Beispiele und Figuren dienen zur näheren Erläuterung der Erfindung, ohne jedoch die Erfindung auf diese Beispiele und Figuren zu begrenzen.

### Beispiele und Figuren:

### Beispiel 1:

### Herstellung von 4'-Hydroxy-Diclofenac durch Leberzellen

HepG2 Zellen (ATCC HB-8065) werden in dem Medium DMEM PAA (Pasching, Austria) kultiviert, welches mit 10 % FCS Gold, 2 mM L-Glutamin, 100 U/ml Penicillin und 0,1mg/ml Streptomycin versetzt wird. Primäre humane Hepatozyten (pHHs) werden kommerziell von z.B. TebuBio (Le Perray-en-Yvelines, Frankreich) oder Promocell (Heidelberg, Deutschland) bezogen. Die Zellen werden auf 50 pg/ml Kollagen Typ I (Rattenschwanz, BD Biosciences, San Jose, USA) beschichtete Zellkulturplatten ausgesät und bei 37°C und 5 % CO2 in Hepatozyten Growth Medium (Promocell GmbH, Heidelberg, Deutschland) oder dem Fachmann bekanntem gleichwertigen Zellkulturmedien anderer Hersteller kultiviert. Diese pHHs können direkt für die Metabolitproduktion eingesetzt werden oder nach Erzeugung proliferationskompetenter Leberzellen. Dafür werden Proliferationsgene mit einem lentiviralen Vektor in die Zellen transduziert. Diese Technik ist in der WO2009/030217A2 (Braspenning et al. (2007)) beschrieben. Zellklone, welche die Proliferationsfähigkeit im Vergleich zu nicht infizierten primären Hepatozyten erhalten haben, wachsen zu in den Zellkulturschalen sichtbaren Kolonien aus und können über Trypsinierung abgelöst und isoliert werden. Die proliferienden Hepatozyten-Klone (Zelllinien) werden in Bezug auf ihre Leberzellmarker charakterisiert. Über quantitative real-time PCR (qRT-PCR) kann nachgewiesen werden, dass die für die Bildung von Phase-I- und Phase-II-Metaboliten notwendigen Enzyme exprimiert werden. Zur Definition von Sequenzen zur Generierung von Primern kann der Fachmann Datenbanken wie NCBI Genbank (http://www.ncbi.nlm.nih.gov/genbank) oder European Nucleotide Archive (http://www.ebi.ac.uk/ena/) verwenden. Für Phase I sind vor allem die Enzyme der Cytochrom- P450 (CYP) Familie relevant. Dazu gehören unter anderem die CYPs 1A2, 2B6, 2C6, 2C8, 2C9, 2C19, 3A4, 3A5, 3A6, 2D5, 2E1. Für Phase II sind u.a. die UDP-Glucuronosyltransfersasen (UGTs), Sulfotransferasen, (STs), N-Acetyltransferasen (NATs), Glutathion-S- Transferasen (GSTs), Methyltransferasen (z.B. TPMT, COMT etc.) relevant. Für alle diese Phase-I- und Phase-II-Enzyme lassen sich geeignete Primer zum Nachweis über qRT-PCR aus Datenbankeinträgen (NCBI, ENA) ableiten. Alternativ kann die Expression von relevanten Enzymen der Biotransformation über Immunfluoreszenz oder Western Blot mittels spezifischer Primärantikörper, welche an die exprimierten Phase-I- und Phase-II-Enzyme binden, nachweisen.

Eine geeignete Hepatozyten-Kultur, ob proliferationsfähige Hepatozyten (z.B. Zellklon HepaFH3) oder nicht proliferationsfähige pHHs werden dann zu z.B. 5x105 Zellen pro well in Kollagen I beschichtete 24-well- Platten ausgesät. Nach 2-7 Tagen in Kultur wird Medium mit 100 µM Diclofenac (Sigma-Aldrich, St. Louis, USA) zu den Zellen gegeben. Die Inkubation findet für 2-24 Stunden im Brutschrank bei 5 % CO2 und 37 °C in Hepatozytenmedium statt. Überstände dieser Zellkulturen werden für Spurenanalytik mit HPLC verwendet. Dazu kann das Gerät: VWR-Hitachi Elite LaChrom series HPLC mit Photodiode array L-2455 (Säule: Phenomenex Kinetex C18, 4.6x150 mm, 5 µm Partikelgröße, 100 Å; Vorsäule: RP18; 4,6x2 mm) verwendet werden. Der Nachweis des gebildeten Metabolits 4'-Hydroxy-Diclofenac kann auch über LC-MS mit einem Waters Alliance HPLC System (Säule: Phenomenex Luna C18[2] 2,0x150 mm, 5 µm Partikelgröße, 100 Å; Vorsäule: RP18; 4x3 mm in Kombination mit einem ZMD SingleQuad-MS Detektionsystem (ESI+; cone voltage: 30eV) durchgeführt werden.

Wie in der Tabelle 1 dargestellt, werden bei HepaFH3 (proliferationsfähige Leberzellen) sowie bei HepG2 (Leberzelllinie) sowie bei primären humanen Hepatozyten (pHH) verschiedener Donoren für das Zellsystem jeweils charakteristische Mengen von Metaboliten gefunden.

**Tabelle 1: Metabolismus von Diclofenac und Testosteron in verschiedenen Leberzellsystemen**

| Substanz Diclofenac | Metabolite 4'-OH-Diclofenac | HepaFH3 (n = 6) | HepG2 (n = 3) | pHH Donor 1 | pHH Donor 2 |
|---|---|---|---|---|---|
| | | 7,13 ± 2,22 | n.n. | 15,3 | 59,4 |
| Testosteron | Androstendion | 84,33 ± 11,59 | 35,32 ± 0,1 | 30,9 | 72,2 |
| | OH-Testosteron | 3,02 ± 1,06 | 2, 76 ± 0,1 | 6,1 | 23,0 |

Die über HPLC und LC-MS Analyse erhaltenen Werte sind in pmol/(min*106 Zellen); n.n. = keine Metabolite nachweisbar; pHH-primäre humane Hepatozyten

### Beispiel 2:

### Herstellung des Phase-II-Metaboliten 7-Hydroxyumarin-Glucuronid aus 7-Ethoxycumarin über ein zweistufiges Verfahren.

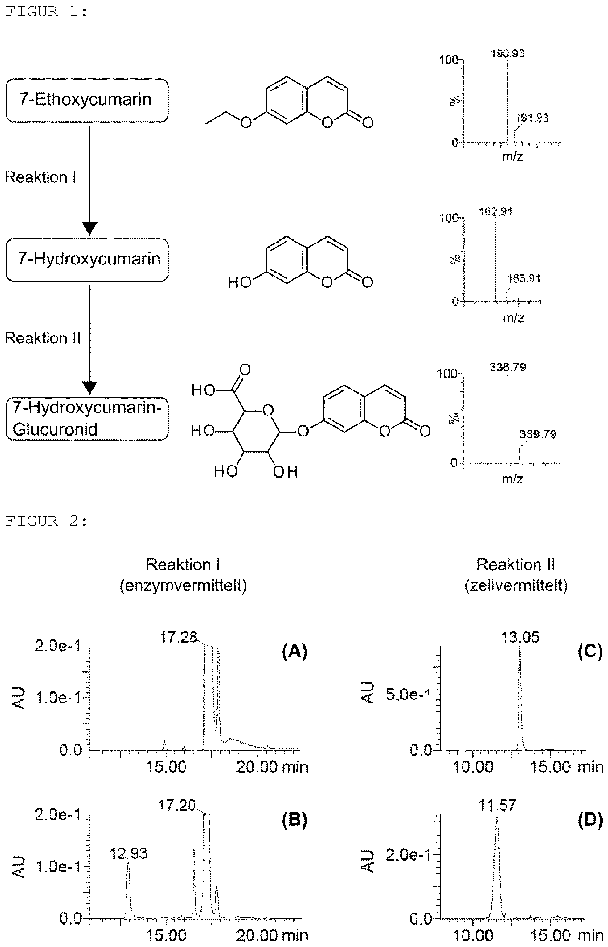

Bei diesem Ausführungsbeispiel wurde die Ausgangssubstanz 7-Ethoxycumarin in einer ersten Reaktion zum Phase-I-Metaboliten 7-Hydroxycumarin katalysiert durch pilzliche Peroxygenasen umgesetzt. In einer Folgereaktion wurde dieses Zwischenprodukt in einem Zell-Bioreaktor zum Phase-II-Metaboliten (Glucuronid) biotransformiert. Diese zweite Reaktion erfolgte an primären oder molekularbiologisch veränderten humanen Leberzellen. Mit diesem Beispiel wird die kombinierte Synthese für humane *First*-*pass*-Arzneimetabolite (Phase I und II) aus einer enzymund zellvermittelten Biotransformation verdeutlicht ((Ullrich *et al.* (2007); Kinne *et al.* (2009); Poraj-Kobielska *et al.* (2011)). In Figur 2 ist das Prinzip der Reaktionsfolge dargestellt.

Für die Reaktion I wurde ein Reaktionsansatz von 0,5-200 ml verwendet (0,5-5 mM Substrat; Peroxygenase des Pilzes *Agrocybe aegerita* mit einer Aktivität von 4-400 Units; Phosphatpuffer 50 mM (pH7); 4 mM Ascorbinsäure). Nach Zugabe des Co-Substrat H₂O₂ (Aktivator der Reaktion; 5 mM) und einer Reaktionszeit von 60 min wurde das gebildete Produkt (7-Hydroxycumarin) *via* LC-MS bestimmt (λmax = 323 nm). In diesem Beispiel handelt es sich somit um eine O-Deethylierungsreaktion von 7-Ethoxycumarin zum Phase-I-Metaboliten 7-Hydroxycumarin. In der zweiten Reaktion werden Zellen (humane Hepatozyten) zur Biotransformation von 7-Hydroxycumarin zum Phase-II-Metaboliten 7-Hydroxycumarin-Glucuronid verwendet (Braspenning *et al.* (2007); Hansen *et al.* (2013); Burkard *et al.* (2012)). Die Hepatozyten wurden in einem Hohlfaser-Bioreaktor (CellFiber-System; Fläche: 75 cm²; Zellzahl: 1x10⁸ Zellen) für mindestens 7 Tage vorinkubiert. Anschließend erfolgte die Applikation des Substrates (100 µM) über einen Mediumswechsel. Die Umsetzung erfolgte im Hepatocyte Growth Medium (Promocell GmbH) über einen Zeitraum von 5-6 Tagen nahezu vollständig (Figur 2).
Die HPLC-Analytik der Substrate und deren Metabolite wurde an einem LC-MS-System (Waters alliance HPLC-System mit ZMD SingleQuad-MS (ESI+; cone voltage: 30eV) unter Verwendung einer RP-HPLC-Säule (Phenomenex Luna C18(2) 2x150mm, 100 Ä, Vorsäule:RP18; 4x3mm) durchgeführt. Erforderliche Quantifizierungen wurden mittels Kalibriergeraden bei verbindungstypischen Absorptionsmaxima (Diclofenac: Amax = 270 nm; Propranolol: Amax = 220 nm) verwendet. Unter Verwendung der Laufmittel A (Wasser + 0,1 % Ameisensäure) und Laufmittel B (Acetonitril) setzte sich der lineare HPLC-Gradient wie folgt zusammen: 5 min (5 % LM B/95 % LM A), von 5 bis 20 min ändert sich das Mischungsverhältnis der Fließmittelzusammensetzung linear auf 100 % Laufmittel B. Die Flussrate betrug 0,5 ml/min (Hansen *et al.* (2013)).

### Beispiel 3

### Herstellung des Phase-II-Metaboliten 2-Hydroxydesipramin β-D-Glucuronid aus Desipramin über ein zweistufiges Verfahren. Kombination aus Leberzellen mit vorhandener Aktivität der Phase-I Biotransformation (primäre Leberzellen oder genetisch veränderte Leberzellen) und rekombinanten Phase-II-Enzym (UDP-Glucuronosyltransferase)

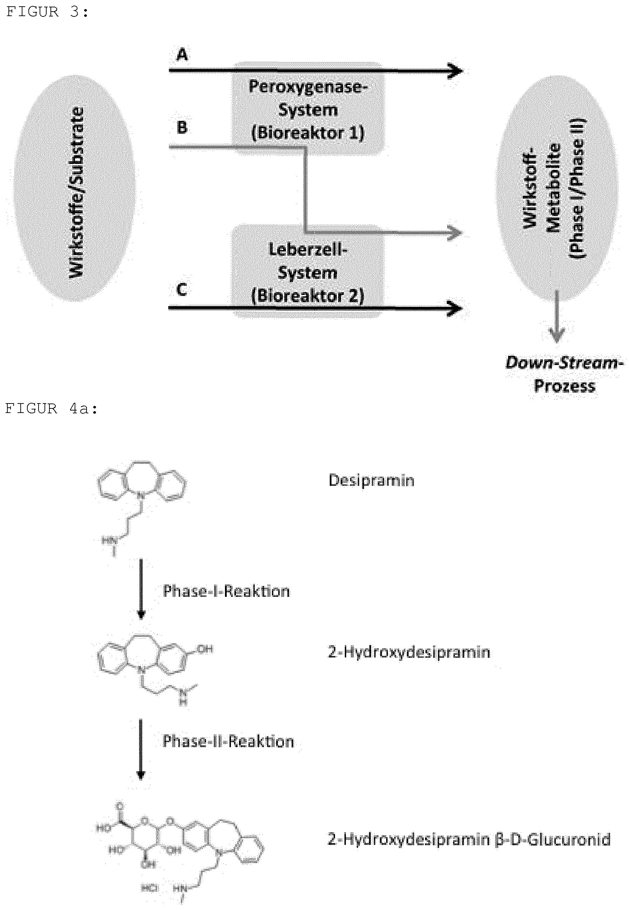

Bei diesem Ausführungsbeispiel wurde die Ausgangsubstanz Desipramin in einer ersten Reaktion zum Phase-I-Metaboliten 2-Hydroxydesipramin katalytisch durch genetisch veränderte humanen Leberzellen umgesetzt. In einer Folgereaktion wurde dieses Zwischenprodukt in einem Zell-Bioreaktor zum Phase-II-Metaboliten (Glucuronid) biotransformiert. Diese zweite Reaktion erfolgte mit rekombinantem funktionsaktiven Phase II Enzym (siehe Figur 4a).

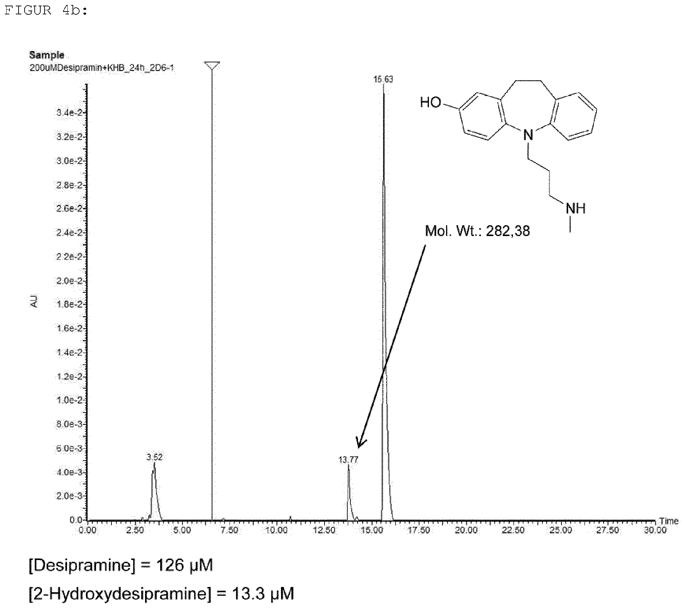

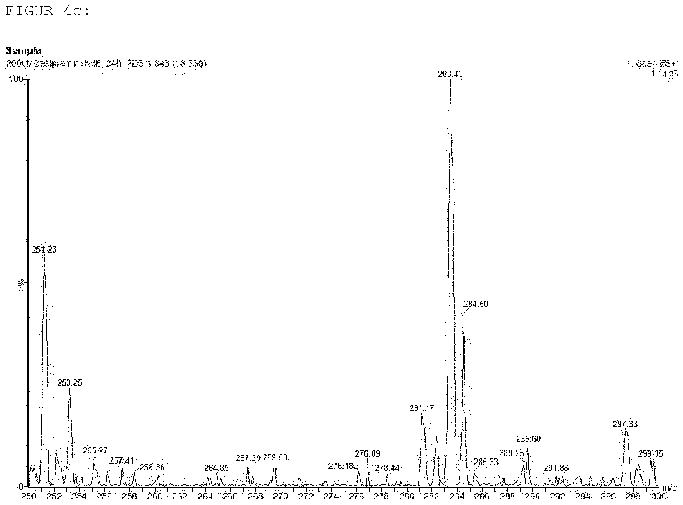

Zunächst wurde ein Reaktionsansatz mit dem Substrat Desipramin von 0,5 ml verwendet (200µM Desipramin in Krebs-Henseleit-Buffer [25mM NaHC03, 2mM CaCl2, 25mM HEPES, pH7,4, Inkubation 24h, 0,5x106 Leberzellen]). Das gebildete Produkt (2-Hydroxydesipramin) wurde via LC-MS bestimmt (λ=254nm; M=282). In diesem Beispiel handelt es um eine Hydroxylierung an Position 2 von Desipramin zu 2-Hydroxydesipramin. In einer zweiten Reaktion erfolgt durch an Membran gebundene rekombinante UDP-Glucuronosyltransferase (erzeugt über E.coli-Expressionssysteme) die Biotransformation von 2-Hydroxydesipramin zum Phase-II-Metaboliten 2-Hydroxydesipramin-β-D-Glucuronid. Der Reaktionsansatz setzt sich aus folgenden Komponenten zusammen: Phosphatpuffer 50 mM (pH7); 0,5 mg/ml UDP-Glucuronosyltransferase; 5 mM UDPGA; 1mM MgCl. Der Nachweis des Phase-II-Metaboliten (2-Hydroxydesipramin-β-D-Glucuronid, M=458) erfolgt via LC/MS (siehe Figur 4b und Figur 4c).

### Beispiel 4

### Kombination aus Peroxygenase-System (gereinigtes Phase-I Enzym) und genetisch veränderte Leberzellen zur Erzeugung von Phase-II Metabolit

Bei diesem Ausführungsbeispiel wurde die Ausgangsubstanz Desipramin in einer ersten Reaktion zum Phase-I-Metaboliten 2-Hydroxydesipramin katalytisch durch pilzliche Peroxygenasen umgesetzt. In einer Folgereaktion wurde dieses Zwischenprodukt in einem Zell-Bioreaktor mit genetisch veränderten Leberzellen zum Phase-II-Metaboliten (Glucuronid) biotransformiert. Mit diesen Beispiel wird die kombinierte Synthese für humane First-pass-Arzneiwirkstoffmetabolite (Phase-I/-II) aus einer enzym- und einer zellvermittelten Biotransformation verdeutlicht.

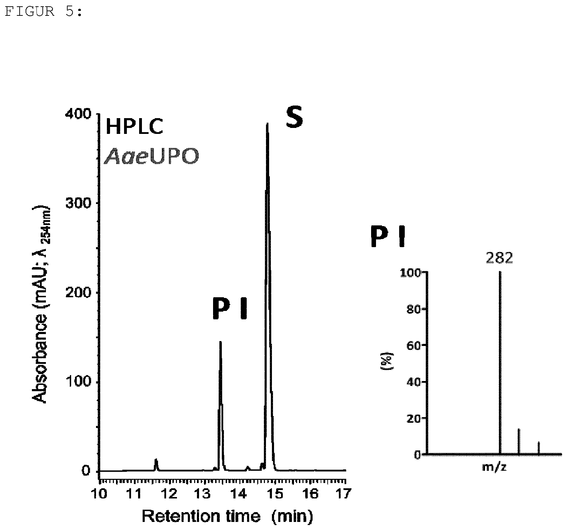

Für die Reaktion I wurde ein Reaktionsansatz von 100 ml verwendet (5 mM Substrat Desipramin; Peroxygenase des Pilzes Agrocybe aegerita mit einer Aktivität von 300 Units; Phosphatpuffer 50 mM (pH7); 4 mM Ascorbinsäure). Nach Zugabe des Co-Substrat H₂O₂ (Aktivator der Reaktion; 5 mM) und einer Reaktionszeit von 60 min wurde das gebildete Produkt (hydroxyliertes Desipramin) via LC-MS bestimmt (λmax = 254 nm). In diesem Beispiel handelt es sich somit um eine Hydroxylierung von Desipramin zum Phase-I-Metaboliten 2-Hydroxydesipramin. In der zweiten Reaktion werden Zellen (genetisch veränderte Leberzellen) zur Biotransformation von 2-Hydroxydesipramin zum Phase-II-Metaboliten 2-2-Hydroxydesipramin-β-D-Glucuronid verwendet. Die Leberzellen wurden in einem Hohlfaser-Bioreaktor (CellFiber-System; Fläche: 75 cm2; Zellzahl: 1x108 Zellen) für 7 Tage vorinkubiert. Anschließend erfolgte die Applikation des Substrates (100 µM) über einen Mediumwechsel. Die Umsetzung erfolgte im Hepatocyte Growth Medium (Promocell GmbH) über einen Zeitraum von 5 Tagen nahezu vollständig. Der Nachweis des Phase-II-Metaboliten (2-Hydroxydesipramin-β-D-Glucuronid, M=458) erfolgt via LC/MS (siehe Figur 5).

### Allgemein für alle Nachweise:

Die HPLC-Analytik der Substrate und deren Metabolite wurde an einem LC-MS-System (Waters alliance HPLC-System mit ZMD SingleQuad-MS (ESI+; cone voltage: 30eV) unter Verwendung einer RP-HPLC-Säule (Phenomenex Kinetex C18(2) 150mmx4,6mm, 100 Å, Vorsäule:RP18; 4x3mm) durchgeführt. Erforderliche Quantifizierungen wurden mittels Kalibriergeraden bei verbindungstypischen Absorptionsmaxima (Diclofenac: Amax = 270 nm; Propranolol: Amax = 220 nm, Desipramin 254 nM) verwendet. Unter Verwendung der Laufmittel A (Wasser + 0,1 % Trifluressigsäure) und Laufmittel B (Acetonitril+0,75 % Trifluressigsäure) setzte sich der lineare HPLC-Gradient wie folgt zusammen: 5 min (5 % LM B/95 % LM A), von 5 bis 20 min ändert sich das Mischungsverhältnis der Fließmittelzusammensetzung linear auf 100 % Laufmittel B. Die Flussrate betrug 1,0 ml/min.

### Nachweis:

i.) LC-MS-System (Waters alliance HPLC system with ZMD SingleQuad-MS (ESI+; cone voltage: 30eV; column: Phenomenex Kinetex C18(2) 250x4,6mm, 5µ particle size, 100 Å; security guard: C18(2), 4x3mm))
ii.) Desipramin (M=266), 2-Hydroxydesipramin (M=282), 2-Hydroxydesipramin β-D-Glucuronid (M=458)

### Beispiel 5

### Herstellung des Phase-II-Metaboliten 5-Hydroxypropranol β-D-Glucuronid aus Propranolol über ein zweistufiges Verfahren.

Bei diesem Ausführungsbeispiel wurde die Ausgangsubstanz Propranolol in einer ersten Reaktion zum Phase-I-Metaboliten 5-Hydroxypropranolol katalytisch durch pilzliche Peroxygenasen umgesetzt. In einer Folgereaktion wurde dieses Zwischenprodukt in einem Zell-Bioreaktor mit genetisch veränderten Leberzellen zum Phase-II-Metaboliten (Glucuronid) biotransformiert. Mit diesem Beispiel wird die kombinierte Synthese für humane First-pass-Arzneiwirkstoffmetabolite (Phase-I/-II) aus einer enzym- und zellvermittelten Biotransformation verdeutlicht.

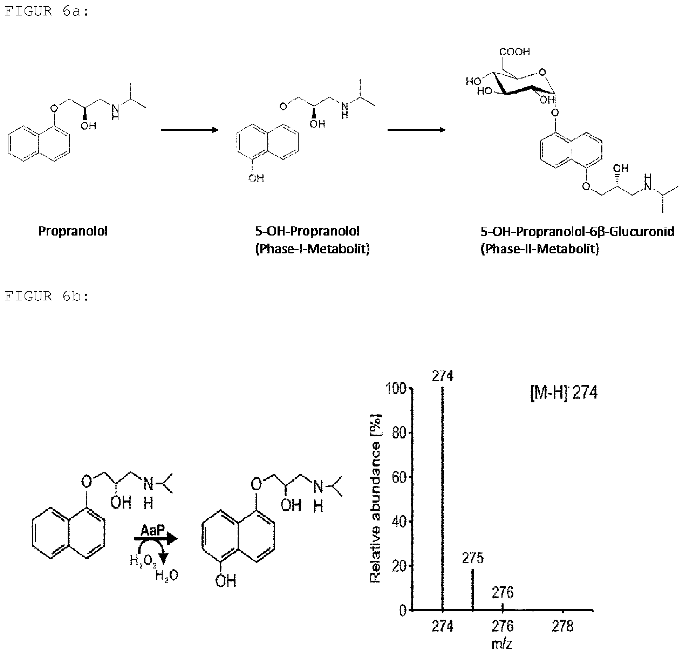

Für die Reaktion I wurde ein Reaktionsansatz 100 ml verwendet (5 mM Substrat Propranolol; Peroxygenase des Pilzes Agrocybe aegerita mit einer Aktivität von 300 Units; Phosphatpuffer 50 mM (pH7); 4 mM Ascorbinsäure). Nach Zugabe des Co-Substrat H₂O₂ (Aktivator der Reaktion; 5 mM) und einer Reaktionszeit von 60 min wurde das gebildete Produkt (hydroxyliertes Propranolol) via LC-MS bestimmt (Amax = 280 nm). In diesem Beispiel handelt es sich somit um eine Ring-Hydroxylierung von Propranolol (M=259) zum Phase-I-Metaboliten 5-Hydroxypropranolol (M=275) (siehe Figur 6a).

In der zweiten Reaktion werden genetisch veränderten Leberzellen zur Biotransformation von 5-Hydroxypropranol zum Phase-II-Metaboliten 5-Hydroxypropranolol-β-D-Glucuronid verwendet. Die Hepatozyten wurden in einem Hohlfaser-Bioreaktor (CellFiber-System; Fläche: 75 cm2; Zellzahl: 1x108 Zellen) für 7 Tage vorinkubiert. Anschließend erfolgte die Applikation des Substrates (Phase I Metabolit, 100 µM) über einen Mediumswechsel. Die Umsetzung erfolgte im Hepatocyte Growth Medium (Promocell GmbH) über einen Zeitraum von 5-6 Tagen nahezu vollständig. Der Nachweis des Phase-II-Metaboliten (5-Hydroxypropranolol-β-D-Glucuronid, M=452) erfolgt via LC/MS (siehe Figur 6b).
**Figur 1****:** Dargestellt ist ein zweistufiges Verfahren zur Umsetzung von dem Substrat 7-Ethoxycumarin über ein Phase-I-Zwischenprodukt (7-Hydroxycumarin) zu einem Phase-II-Metaboliten (7-Hydroxycumarin-Glucuronid). Reaktion I (Modul pilzliche Peroxygenase): Grundlage sind extrazelluläre Enzyme pilzlichen Ursprungs (Peroxygenase; *Agrocybe aegerita* [1, 2]; Reaktion II (Modul Hepatozyten): In einer Folgereaktion wird der Phase-II-Metabolit (7-Hydroxycumarin-Glucuronid) mit einem präparativen 3D-Zellkulturverfahren (Hohlfasermodul-System, Fibercell Inc.) mit veränderten humanen Leberzellen (Hepatozyten) generiert.
**Figur 2****:** HPLC-Chromatogramme (λₘₐₓ=323nm) : Umsetzung von 7-Ethoxycumarin (ret. time = 17,2 min) zu 7-Hydroxycumarin (ret. time = 12,9 min) mit fungaler Peroxygenase ((A) Reaktionszeit: 0 h; (B) Reaktionszeit: 0,5 h) in der ersten Reaktion. In der zweiten Folgereaktion ist die Biotransformation von 7-Hydroxycumarin (ret. time = 13,0 min) zum Phase-II-Metabolit 7-Hydroxycumarin-Glucuronid (ret. time = 11,6 min) mit humanen Hepatozyten dargestellt ((C) Reaktionszeit: 0 h; (D) Reaktionszeit: 24 h).
**Figur 3****:** Darstellung des Erfindungsprinzips.
**Figur 4a****:** Reaktionsfolge der selektiven Umsetzung von Desipramin.
**Figur 4b****:** HPLC-Chromatogramm Umsetzung mit Desipramin zum Phase I Metabolit mit genetisch veränderten Leberzellen.
**Figur 4c****:** Massenspektrum zur Demonstration der Umsetzung von Desipramin mit genetisch veränderten Leberzellen.
**Figur 5****:** Links, HPLC-Chromatogramm zur Umsetzung Desipramin mit Peroxygenase-System (AaeUPO = Agrocybe aegerita Peroxygenase, S=Substrat, Desipramin, P1 = Produkt, hydroxyliertes Desipramin, M=282), rechts LC-MS Massenspektrum des Produkts.
**Figur 6a****:** Reaktionsfolge der selektiven Umsetzung von Propranolol.
**Figur 6b****:** Massenspektrum zur Umsetzung von Propranolol mit Peroxygenase-Enzym-System zu Phase-I-Metabolit (Kinne et al. 2009) .

### Zit. Literatur:

1. Elaut G, Henkens T, Papeleu P, Snykers S, Vinken M, Vanhaecke T, Rogiers V (2006): Molecular mechanisms underlying the dedifferentiation process of isolated hepatocytes and their cultures. Current drug metabolism 7 (6): 629-60.
2. Arimoto R (2006): Computational models for predicting interactions with cytochrome p450 enzyme. Current topics in medicinal chemistry 6 (15): 1609-18.
3. Shimada T, Yamazaki H, Mimura M, Inui Y, Guengerich FP (1994): Interindividual variations in human liver cytochrome P-450 enzymes involved in the oxidation of drugs, carcinogens and toxic chemicals: studies with liver microsomes of 30 Japanese and 30 Caucasians. The Journal of pharmacology and experimental therapeutics 270 (1): 414-23.
4. Lamb DC, Waterman MR, Kelly SL, Guengerich FP (2007): Cytochromes P450 and drug discovery. Current opinion in biotechnology 18 (6): 504-12.
5. Chang CY, Schiano TD (2007): Review article: drug hepatotoxicity. Alimentary pharmacology & therapeutics 25 (10): 1135-51.
6. Walker K, Ginsberg G, Hattis D, Johns DO, Guyton KZ, Sonawane B (2009): GENETIC POLYMORPHISM IN N-ACETYLTRANSFERASE (NAT): POPULATION DISTRIBUTION OF NAT1 AND NAT2 ACTIVITY. Journal of Toxicology and Environmental Health-Part B-Critical Reviews 12 (5-6): 440-472
7. Ullrich R, Kinne M, Hofrichter M, Scheibner K (2007): Verfahren zur O-Dealkylierung von Alkyarylethern. DE 10 2007 058 741.6.
8. Kinne M, Poraj-Kobielska M, Aranda E, Ullrich R, Hammel KE, Scheibner K, Hofrichter M (2009): Regioselective preparation of 5-hydroxypropranolol and 4'-hydroxydiclofenac with a fungal peroxygenase. Bioorganic & medicinal chemistry letters 19 (11): 3085-7.
9. Poraj-Kobielska M, Kinne M, Ullrich R, Scheibner K, Kayser G, Hammel KE, Hofrichter M (2011): Preparation of human drug metabolites using fungal peroxygenases. Biochemical pharmacology 82 (7): 789-96.
10. Hansen M, Herzog N, Miethbauer S, Schmidtke KU, Lupp A, Sperling S, Seehofer D, Damm G, Scheibner K, Küpper J-H (2013): Primary-like human hepatocytes genetically engineered to obtain proliferation competence display hepatic differentiation characteristics in monolayer and organotypical spheroid cultures. Drug metabolism and disposition: the biological fate of chemicals (submitted).
11. Burkard A, Dahn C, Heinz S, Zutavern A, Sonntag-Buck V, Maltman D, Przyborski S, Hewitt NJ, Braspenning J (2012): Generation of proliferating human hepatocytes using Upcyte(R) technology: characterisation and applications in induction and cytotoxicity assays. Xenobiotica; the fate of foreign compounds in biological systems 42 (10): 939-56.

## Patentansprüche

1. Verfahren zur Herstellung von biogenen Stoffen in mindestens einem Bioreaktor, **dadurch gekennzeichnet, dass**
a) mindestens ein Ausgangsstoff mit
b) mindestens einem Enzym versetzt wird und das erhaltene Produkt mit
c) mindestens einer Leberzelle versetzt wird,
oder
b') mindestens einer Leberzelle versetzt wird und das erhaltene Produkt mit
c') mindestens einem Enzym versetzt wird,
und
d) mindestens ein biogener Stoff isoliert wird,
wobei der Ausgangstoff mindestens eine chemische Substanz enthält und mindestens ein Enzym ausgewählt ist aus der Gruppe Phase-I-Enzyme, Phase-II-Enzyme und Oxidoreduktasen.

2. Verfahren nach Anspruch 1, **dadurch gekennzeichnet, dass** der Ausgangstoff mindestens eine chemische Substanz enthält ausgewählt aus der Gruppe Arznei- oder Wirkstoff, organische Moleküle, insbesondere mit weniger als 1.000 g/mol.

3. Verfahren nach Anspruch 1 oder 2, **dadurch gekennzeichnet, dass** das Enzym aus b) und c') im Gegensatz zu der Leberzelle aus einem anderen Organismus stammt.

4. Verfahren nach einem der vorhergehenden Ansprüche, wobei mindestens ein Bioreaktor, vorzugsweise zwei oder mehrere Bioreaktoren zur Ausführung der Verfahrensschritte b) (b') und c) (c') eingesetzt werden, und mindestens ein Schritt b) (b') und c) (c') auch wiederholt oder nachgeschaltet werden kann.

5. Verfahren nach einem der vorhergehenden Ansprüche, wobei das Enzym ausgewählt ist aus Oxidoreduktasen, und zwar Monooxygenasen, Dioxygenasen, Oxidasen, Dehydrogenasen, Reduktasen und Peroxygenasen.

6. Verfahren nach einem der vorhergehenden Ansprüche, wobei die Leberzelle ausgewählt ist aus einer Zelle enthaltend mindestens Enzyme zur Biotransformation, insbesondere Phase-I-Enzyme und/oder Phase-II-Enzyme, humane Leberzelle, Säugerleberzelle, genetisch veränderte Hepatozyten, proliferierenden Leberzellen, Leberzelllinien.

7. Verfahren nach einem der vorhergehenden Ansprüche, wobei Phase I Enzyme Cytochrom P450 System, Alkohol Dehydrogenasen, Aldehyd Dehydrogenasen, Peroxidasen, Glutathionperoxidase, Esterasen, Hydrolasen umfassen und/oder Phase 1I Enzyme Glucuronyltransferasen, Sulfotransferasen, Glutathion-S-Transferase, Methyltransferasen, Aminotransferasen / Transaminasen, Acetyltransferasen umfassen.

8. Verfahren nach einem der vorhergehenden Ansprüche, **dadurch gekennzeichnet, dass** der isolierte biogene Stoff ein organisches Molekül ist, welches neben Kohlenstoff und Wasserstoff Heteroatome enthalten kann und weniger als 1.000 g/mol aufweist.

9. Verfahren nach einem der vorhergehenden Ansprüche, **dadurch gekennzeichnet, dass** der mindestens eine Bioreaktor ein Medium, Kulturmedium oder Kulturflüssigkeit enthält.

10. Verfahren nach einem der vorhergehenden Ansprüche, **dadurch gekennzeichnet, dass** der mindestens eine Bioreaktor einen Rührer enthält.

11. Verfahren nach einem der vorhergehenden Ansprüche, **dadurch gekennzeichnet, dass** der mindestens eine Bioreaktor ausgewählt ist aus der Gruppe Hohlfaser-Bioreaktoren, Rührkesselreaktor, Fermenter, Wirbelschichtreaktor, Festbettreaktoren mit Hohlfaser- bzw. Flachbettmembranen, Plug-Flow-Reactor und Spinner-Flasche.

## Claims

1. A process to produce biogenic substances in at least one bioreactor, **characterized in that**
a) at least one starting material with
b) at least one enzyme is added and the obtained product with
c) at least one liver cell is added,
or
b') at least one liver cell is added and the obtained product with
c') at least one enzyme is added,
and
d) at least one biogenic substance is isolated.
wherein the starting material contains at least one chemical substance and at least one enzyme is selected from the group of phase I enzymes, phase II enzymes and oxidoreductases.

2. The process according to claim 1, **characterized in that** the starting material contains at least one chemical substance selected from the group of drug or active compound, organic molecules, in particular less than 1.000 g/mol.

3. The process according to claim 1 or 2, **characterized in that** the enzyme from b) and c') comes from a different organism than the liver cell does.

4. The process according to one of the preceding claims,
wherein at least one bioreactor, preferably two or more bioreactors, are used to carry out process steps b) (b') and c) (c'), and at least one step b) (b') and c) (c') can also be repeated or downstream.

5. The process according to one of the preceding claims,
wherein the enzyme is selected from the oxidoreductases, namely monooxygenases, dioxygenases, oxidases, dehydrogenases, reductases, and peroxygenases.

6. The process according to one of the preceding claims,
wherein the liver cell is selected from a cell containing at least biotransformation enzymes, in particular phase I enzymes and/or phase II enzymes, a human liver cell, a mammalian liver cell, genetically modified hepatocytes, proliferating liver cells, and liver cell lines.

7. The process according to one of the preceding claims,
wherein phase I enzymes comprise the cytochrome P450 system, alcohol dehydrogenases, aldehyde dehydrogenases, peroxidases, glutathione peroxidase, esterases, hydrolases and/or phase II enzymes comprise glucuronyltransferases, sulfotransferases, glutathione S-transferase, methyltransferase, aminotransferases / transaminases, and acetyltransferases.

8. The process according to one of the preceding claims, **characterized in that** the isolated biogenic substance is an organic molecule, which may contain besides carbon and hydrogen, heteroatoms and having less than 1.000 g/mol.

9. The process according to one of the preceding claims, **characterized in that** the at least one bioreactor contains medium, culture medium or culture fluid.

10. The process according to one of the preceding claims, **characterized in that** the at least one bioreactor contains a stirrer.

11. The process according to one of the preceding claims, **characterized in that** the at least one bioreactor is selected from the group of hollow fiber bioreactors, stirred tank reactors, fermenters, fluidized-bed reactors, fixed-bed reactors with hollow fiber or flat membrane beds, plug-flow reactors and spinner bottles.

## Revendications

1. Procédé de fabrication de substances biogènes dans au moins un bioréacteur, caractérisé en ce
a) qu'au moins une substance de départ est mise à réagir avec
b) au moins une enzyme et que la substance obtenue est mise à réagir avec
c) au moins une cellule hépatique,
ou
b') qu'au moins une cellule hépatique est mise à réagir et que la substance obtenue est mise à réagir avec
c') au moins une enzyme,
et
d) au moins une substance biogène est isolée,
la substance de départ contenant au moins une substance chimique, et au moins une enzyme est sélectionnée dans le groupe d'une enzyme phase de I, d'une enzyme de phase II et des oxydoréductases.

2. Procédé selon la revendication 1, **caractérisé en ce que** la substance de départ contient au moins une substance chimique du groupe des matières médicamenteuses ou actives, des molécules organiques, notamment en quantité inférieure à 1000 g/mol.

3. Procédé selon la revendication 1 ou la revendication 2, **caractérisé en ce que** l'enzyme provenant de b) et de c') provient d'un autre organisme, contrairement à la cellule hépatique.

4. Procédé selon l'une des revendications précédentes, dans lequel au moins un bioréacteur, de préférence, deux ou plusieurs bioréacteurs, sont employés pour l'exécution des étapes de procédé b), (b') et c), (c'), et au moins une étape b), (b') et c), (c') peut être également répétée ou rajoutée.

5. Procédé selon l'une des revendications précédentes, dans lequel l'enzyme est choisie parmi des oxydoréductases, et en l'occurrence, des mono oxygénases, des di oxygénases, des oxydases, des déshydrogénases, des réductases et des peroxygénases.

6. Procédé selon l'une des revendications précédentes, dans lequel la cellule hépatique est choisie à partir d'une cellule contenant au moins des enzymes pour la biotransformation, notamment, des enzymes de phase I et/ou des enzymes de phase II, la cellule hépatique humaine, la cellule hépatique de mammifère, des hépatocytes génétiquement modifiés, des cellules hépatiques proliférantes, des lignées cellulaires hépatiques.

7. Procédé selon l'une des revendications précédentes, dans lequel les enzymes de phase I comprennent le système cytochrome P450, des alcools déshydrogénases, des aldéhydes déshydrogénases, des peroxydases, le glutadion peroxydase, des estérases, des hydrolases et/ou des enzymes de phase II comprennent des glucuronyl transférases, des sulfo transférases, la glutadion S transférase, des méthyl transférases, des amino transférases/ transaminases, des acétyl transférases

8. Procédé selon l'une des revendications précédentes, **caractérisé en ce que** la substance biogène est une molécule, laquelle peut contenir, à côté du carbone et de l'hydrogène, des hétéroatomes et représente moins de 1000 g/mol.

9. Procédé selon l'une des revendications précédentes, **caractérisé en ce que** l'au moins un bioréacteur contient un milieu, un milieu de culture ou un liquide de culture.

10. Procédé selon l'une des revendications précédentes, **caractérisé en ce que** l'au moins un bioréacteur contient un agitateur.

11. Procédé selon l'une des revendications précédentes, **caractérisé en ce que** l'au moins un bioréacteur est choisi dans le groupe des bioréacteurs en fibres creuses, d'un réacteur type cuve agité, d'un fermenteur, d'un réacteur à lit fluidisé, des réacteurs à lits fixes avec des membranes à fibres creuses, respectivement, des membranes pour lits plans, d'un réacteur plug-flow et d'un flacon type spinner.
